# EUROPEAN PATENT APPLICATION

(11) **EP 4 810 403 A1**
(43) Date of publication of application: **23.09.2026**
(21) Application number: 24891240.4
(22) Date of filing: 31.10.2024
(51) Int. Cl.: B65D 81/30, A61J 1/00, B65D 77/00, B65D 83/00

(54) **LIQUID AGENT CONTAINER AND APPLICATOR**

(30) Priority: 16.11.2023 JP 2023194938
(71) Applicant: Kaken Pharmaceutical Co., Ltd., Tokyo 113-8650 (JP)
(72) Inventor: NATORI, Nobuyuki, Fujieda-shi, Shizuoka 426-8646 (JP); TAKABE, Hiroyuki, Fujieda-shi, Shizuoka 426-8646 (JP); TAKEI, Ryoji, Soka-shi, Saitama 340-0017 (JP); KONAGAI, Runa, Soka-shi, Saitama 340-0017 (JP)
(74) Representative: Hoffmann Eitle
(86) International application number: PCT/JP2024/038905
(87) International publication number: WO 2025/105199

(57) **Abstract**

The present invention provides a liquid agent container for loading a liquid agent of efinaconazole, the liquid agent container having two conflicting features of enabling the quantity of a liquid agent to be visually recognized from the outside while holding photostability. A liquid agent container into which a liquid agent containing 10% of efinaconazole can be loaded, wherein the liquid agent container is a container formed from a synthetic resin containing titanium (IV) oxide, a transmittance for light having a wavelength of 200 nm to 360 nm is all 0.20% or less, and a transmittance for light having a wavelength of 700 nm is 0.25% or more, and a quantity of the liquid agent loaded into the liquid agent container is visually recognizable from the outside.

## Description

### TECHNICAL FIELD

The present invention relates to a liquid agent container for loading a liquid agent containing efinaconazole. In addition, the present invention relates to an applicator having the liquid agent container.

### BACKGROUND ART

Efinaconazole is a triazole-based compound having an antifungal activity represented by the following formula (I):

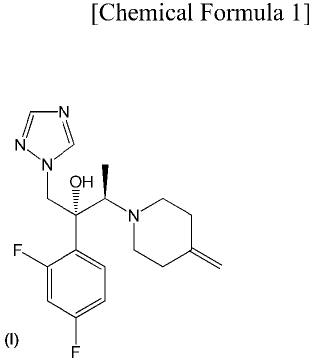

Efinaconazole is known as an active ingredient of a topical tinea unguium therapeutic agent. A liquid agent containing efinaconazole is on sale under the product name of CLENAFINE (Registered Trademark) topical solution 10% in Japan and under the product name of JUBLIA (Registered Trademark) topical solution, 10% in the USA (NPL 1).

In liquid agents containing efinaconazole, an increase in the amount of efinaconazole analogues are recognized in a photostability stress test. Therefore, when a container enabling the remaining amount of a liquid agent to be visually recognized from the outside easily (for example, a colorless and transparent glass container) is used, the degradation of efinaconazole is accelerated. Therefore, it is extremely important to secure the stability of a formulation against light exposure. Therefore, conventional efinaconazole formulations are loaded into white opaque light-resistant containers. However, detailed insights on the photostability of efinaconazole are yet not known.

In the case of using a liquid agent containing efinaconazole for a long period of time in the treatment of tinea unguium, the administration period lasts for several months or longer. A patient preferably goes to the doctor before taking the entire liquid agent. Therefore, from the viewpoint of improvement in the patient adherence (in other words, medication compliance), it is important to enable the remaining amount of the liquid agent to be visually recognized from the outside. However, conventional containers for efinaconazole formulations are white opaque light-resistant containers, and normally, it is thus impossible to visually recognize the quantity of liquid agents from the outside.

Efinaconazole is used as a liquid agent that is topically applied to nails. Therefore, an applicator in which a liquid agent container for loading a medicine and a brush member for applying the liquid agent to nails are integrated together is suitable. As such an application container with a brush, for example, an applicator for applying a tinea unguium therapeutic agent to an infected area has been disclosed in PTL 1. As an example of a liquid agent container (that is, a bottle portion) configuring the applicator, a liquid agent container formed of an organic material and capable of containing a liquid medicine has been disclosed. Examples of the organic material include polyolefins such as polyethylene and polypropylene, aromatic polyesters such as polyethylene terephthalate and polybutylene terephthalate, and the like.

However, in the publication, there are no descriptions of efinaconazole, let alone any disclosure of a liquid agent container into which efinaconazole is loaded and which is capable of stably containing efinaconazole.

Hitherto, there have been a variety of proposals of medical containers enabling the quantity of a liquid agent to be visually recognized from the outside and capable of preventing deterioration of an active ingredient by light.

PTL 2 discloses an ultraviolet-shielding plastic medical container containing 0.01 to 1.00 wt% of titanium oxide having an average particle size of 10 to 40 µm added to a thermoplastic resin.

PTL 3 discloses a pharmaceutical formulation in a transparent package containing a pharmaceutical formulation containing tranilast or a salt thereof in a transparent package provided with light shielding means for shielding rays of light having a wavelength of 350 to 450 nm.

PTL 4 discloses a container for which a colorant-containing colored transparent resin is used, the colorant-containing colored transparent resin having a transmittance of 10% or less for light having a wavelength of 200 nm to 500 nm and a transmittance of 80% or more for light having a wavelength of 540 nm to 800 nm.

However, the containers described in PTL 2 to 4 are not applicators for applying a liquid agent to nails. In addition, in the container described in PTL 2, it seems that deterioration of contents occurs based on the description of the publication. The containers described in PTL 3 and 4 are also not necessarily capable of fully shielding the rays of light in the ultraviolet region.

### CITATION LIST

### PATENT LITERATURE

PTL 1: International Publication No. WO 2013/005434
PTL 2: Japanese Patent Application Laid-Open No. H8-98870
PTL 3: Japanese Patent Application Laid-Open No. 2014-015467
PTL 4: Japanese Patent Application Laid-Open No. 2007-061192

### NON PATENT LITERATURE

NPL 1: CLENAFINE (Registered Trademark) topical solution 10% Interview Form revised July 2022 (8^{th} edition)

### SUMMARY OF INVENTION

### TECHNICAL PROBLEM

One object that the present invention is intended to achieve is to provide a liquid agent container for loading a liquid agent of efinaconazole, the liquid agent container having two conflicting features of enabling the quantity of a liquid agent to be visually recognized from the outside while holding photostability.

Another object that the present invention is intended to achieve is to provide an application container with a brush (in other words, an applicator) for loading and applying a liquid agent of efinaconazole, the application container with a brush enabling the quantity of a liquid agent to be visually recognized from the outside while holding photostability and enabling the application of an appropriate amount of a liquid agent.

### SOLUTION TO PROBLEM

In order to achieve the above-described objects, the present inventors studied the photostability of efinaconazole in detail. As a result, it was found that efinaconazole is extremely susceptible to the rays of light in the ultraviolet region and yellowing over time and/or an increase in the amount of analogues is recognized. In addition, it has been clarified that even when the transmittance of a container for light in the ultraviolet region is, for example, approximately 1%, degradation of efinaconazole is accelerated.

As described above, as containers for liquid agents suppressing the transmission of the rays of light in the ultraviolet region and enabling the remaining amount of a liquid agent to be confirmed, there have been thus far a variety of proposals. However, none of them are liquid agent containers or applicators for applying a liquid agent to nails. In addition, the fact that efinaconazole is extremely susceptible to the rays of light in the ultraviolet region has made it impossible to use the containers proposed in the art as containers for efinaconazole formulations.

As a result of the present inventors' studies, it has been clarified that a container having two conflicting features of enabling a liquid agent to be visually recognized from the outside while holding the photostability of efinaconazole can be realized by elaborately controlling the light transmittance of the container for light both in the ultraviolet region and in the visible light region.

Specifically, the present inventors found that when a container having a light transmittance for the rays of light in the ultraviolet regions controlled to be within a range of 0% to 0.20% is used, the photostability of efinaconazole is maintained. In addition, the present inventors found that in order to enable the remaining amount of a liquid agent to be visually recognized, the light transmittance for the rays of light having specific wavelengths in the visible light regions needs to be 0.25% or more.

Based on the above-described insights, the present inventors further conducted detailed studies to find a container suitable for the loading of a liquid agent containing efinaconazole.

As described above, as a container suitable for a tinea unguium therapeutic agent, an applicator including a substantially cylindrical container with an opening portion, in which a columnar brush member shaped in a columnar shape by bundling synthetic fibers is installed, has been proposed (PTL 1). The brush member functions as an application member, and a user is able to apply a liquid agent to nails by inverting the container to permeate the liquid agent into the brush member. However, when the container has been inverted for use, there is a case where the liquid agent is excessively discharged or only a small amount of the liquid agent is discharged depending on the usage environment or the design of the container. Therefore, smooth application of the liquid agent is hindered in some cases.

Therefore, in order to realize a preferable sensation of use of the application container with a brush, which is intended to apply a liquid agent to nails, for example, the material, hardness, thickness, and/or the like of the container needs to be designed as appropriate so that an appropriate amount of a liquid agent is rapidly ejected from the container in actual usage. Meanwhile, since the thickness of the container also affects the light transmittance, it is difficult to design a container solving a variety of problems including photostability or visibility in a well-balanced manner.

In view of what has been described above, the present inventors further repeated studies and consequently found that an excellent container for which a variety of problems of photostability, visibility, and a sensation of use as an application container with a brush have been comprehensively solved can be obtained.

As a result of the above-described studies, according to one embodiment of the present invention, it is possible to provide a suitable liquid agent container as a liquid agent container of an applicator for applying a liquid agent to nails. In addition, according to one embodiment of the present invention, it is possible to provide an applicator having a columnar brush member shaped in a columnar shape by bundling synthetic fibers installed in an opening portion of the liquid agent container. The applicator including the liquid agent container according to one embodiment of the present invention is capable of rapidly ejecting an appropriate amount of a liquid agent when inverted in an actual usage and thus has an excellent sensation of use.

In addition, for the applicator according to one embodiment of the present invention, in a case where 4 mL of an efinaconazole 10% solution in ethanol has been loaded into the applicator, and the applicator has been inverted in an environment of 32°C, the number of the drops of the liquid agent dropped until the liquid agent does not drop any more for one minute is 7 to 10.

As described above, the present inventors studied a liquid agent container suitable for loading a liquid agent of efinaconazole. As a result, the present inventors found a container having an extremely favorable sensation of use in which the photostability of an active ingredient of a liquid agent is guaranteed and the remaining amount of the liquid agent is visually recognizable and completed the present invention.

That is, the present invention is as follows:
[1] A liquid agent container into which a liquid agent containing 10% of efinaconazole can be loaded,
   wherein the liquid agent container is a container formed from a synthetic resin containing titanium (IV) oxide,
   transmittance for light having a wavelength of 200 nm to 360 nm is all 0.20% or less, transmittance for light having a wavelength of 700 nm is 0.25% or more, and a quantity of the liquid agent loaded into the liquid agent container is visually recognizable from outside.
[2] The liquid agent container according to [1], wherein the liquid agent container is a substantially cylindrical polyethylene container having an opening portion in an upper portion, and the container is composed of a single layer containing at least titanium (IV) oxide uniformly dispersed in the polyethylene and is an inflexible and hard container.
[3] The liquid agent container according to [1] or [2], wherein the liquid agent container has an internal volume of 8 mL to 12 mL and a thickness of 0.8 mm to 1.0 mm in a side wall of the liquid agent container.
[4] The liquid agent container according to any one of [1] to [3], wherein a content of the titanium (IV) oxide is 0.09 to 0.33 parts by weight relative to 100 parts by weight of the synthetic resin.
[5] The liquid agent container according to any one of [1] to [4], wherein a content of the titanium (IV) oxide is 0.10 to 0.30 parts by weight relative to 100 parts by weight of the synthetic resin.
[6] The liquid agent container according to any one of [1] to [5], wherein a content of the titanium (IV) oxide is 0.18 to 0.22 parts by weight relative to 100 parts by weight of the synthetic resin.
[7] The liquid agent container according to any one of [1] to [6], wherein no coloring ingredients other than the titanium (IV) oxide are contained.
[8] The liquid agent container according to any one of [1] to [7], wherein transmittance of the liquid agent container for light having wavelengths of 700 nm to 780 nm is all 0.25% or more.
[9] The liquid agent container according to any one of [1] to [8], wherein transmittance of the liquid agent container for light having wavelengths of 700 nm to 780 nm is all 0.40% or more.
[10] The liquid agent container according to any one of [1] to [9], wherein transmittance of the liquid agent container for light having wavelengths of 200 nm to 360 nm is all 0.10% or less.
[11] The liquid agent container according to any one of [1] to [10], wherein the liquid agent container is not packaged with an ultraviolet absorbing film.
[12] An applicator comprising:
   the liquid agent container according to any one of [1] to [11];
   a columnar brush member shaped in a columnar shape by bundling synthetic fibers; and
   a bottomed tubular holder having a tubular main body and a bottom portion between the liquid agent container and the columnar brush member,
   wherein the bottomed tubular holder is liquid-tightly fitted into the opening portion of the liquid agent container,
   the bottom portion of the bottomed tubular holder has at least one pore,
   the tubular brush member is inserted into an inside of the tubular main body of the bottomed tubular holder, and
   the liquid agent is allowed to pass from the liquid agent container to the tubular brush member through the pore, which makes it possible, when the liquid agent container is inverted during use of the applicator, for the liquid agent to be permeated into the tubular brush member and be applied to a user's nail.
[13] The applicator according to [12], wherein the tubular brush member is shaped by bundling a synthetic fiber having a fiber diameter within a range of 7 µm to 50 µm at a density within a range of 0.25 to 0.50, and
   the bottomed tubular holder has one circular pore having a diameter of 0.9 mm to 1.3 mm.
[14] The applicator according to [12] or [13], wherein in a case where the applicator loaded with 4 mL of the liquid agent is inverted in an environment of 32°C, the number of drops dropped until the liquid agent does not drop any more for one minute is 7 to 10.
[15] A photostabilization method of efinaconazole, comprising: a step of loading a liquid agent containing 10% of efinaconazole into the liquid agent container according to any one of [1] to [11] or the applicator according to any one of [12] or [14].

### ADVANTAGEOUS EFFECTS OF INVENTION

Since the use of the liquid agent container according to one embodiment of the present invention suppresses the photolysis of efinaconazole, it is possible to stably preserve a liquid agent of efinaconazole for a long period of time. Furthermore, a user is able to visually recognize the remaining amount of the liquid agent loaded into the liquid agent container from the outside. In addition, it is possible to configure an applicator by installing a columnar brush member shaped in a columnar shape by bundling synthetic fibers in the opening portion of the liquid agent container having the above-described features. The applicator is capable of rapidly ejecting the liquid agent when inverted in an actual usage, drops an appropriate amount of the liquid agent, and thus has a preferable sensation of use.

### BRIEF DESCRIPTION OF DRAWINGS

Fig. 1 is a schematic front view showing one example of an applicator including a liquid agent container according to one embodiment of the present invention.
Fig. 2 is a schematic cross-sectional view of the applicator shown in Fig. 1, the applicator including a cap that covers a columnar brush member.
Fig. 2A is a schematic cross-sectional view for describing the liquid agent container shown in Fig. 1.
Fig. 2B is a schematic perspective view for describing the columnar brush member shown in Fig. 1.
Fig. 2C is a schematic cross-sectional view for describing a bottomed tubular holder shown in Fig. 1.
Fig. 3 is a light transmittance spectrum of a container of Comparative Example 1 at wavelengths of 200 nm to 800 nm.
Fig. 4 is a light transmittance spectrum obtained by enlarging the vertical axis in Fig. 3.
Fig. 5 is a light transmittance spectrum of a container of Comparative Example 2 at wavelengths of 200 nm to 800 nm.
Fig. 6 is a light transmittance spectrum of a container of Comparative Example 3 at wavelengths of 200 nm to 800 nm.
Fig. 7 is a light transmittance spectrum of a container of Comparative Example 4 at wavelengths of 200 nm to 800 nm.
Fig. 8 is a light transmittance spectrum of a container of Example 1 at wavelengths of 200 nm to 800 nm.
Fig. 9 is a light transmittance spectrum obtained by enlarging the vertical axis in Fig. 8.
Fig. 10 is a light transmittance spectrum of a container of Example 2 at wavelengths of 200 nm to 800 nm.
Fig. 11 is a light transmittance spectrum obtained by enlarging the vertical axis in Fig. 10.
Fig. 12 is a light transmittance spectrum of a container of Example 3 at wavelengths of 200 nm to 800 nm.
Fig. 13 is a light transmittance spectrum of a container of Example 4 at wavelengths of 200 nm to 800 nm.
Fig. 14 is a light transmittance spectrum of a container of Example 5 at wavelengths of 200 nm to 800 nm.

### DESCRIPTION OF EMBODIMENTS

Hereinafter, each term in the present specification will be described. In a case where a numerical range is expressed using "to" in the present specification, the numerical range is considered to include the numerical values at both ends.

In the present specification, "ultraviolet region" refers to light having wavelengths of 200 nm to 360 nm, and "visible light region" refers to light having wavelengths of 400 nm to 780 nm.

### <1> Container

In the present specification, a liquid agent container will be simply referred to as "container" in some cases. In addition, the liquid agent container equipped with a columnar brush member will be, as a whole, referred to as "application container with a brush" or "applicator" in some cases.

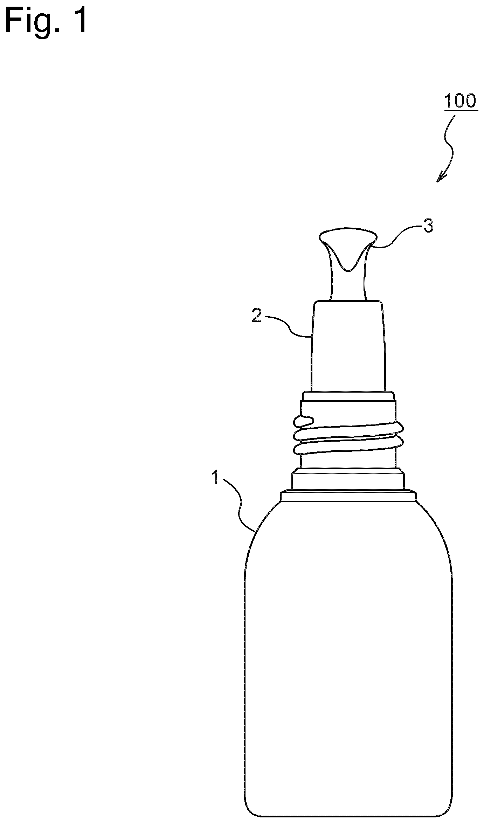

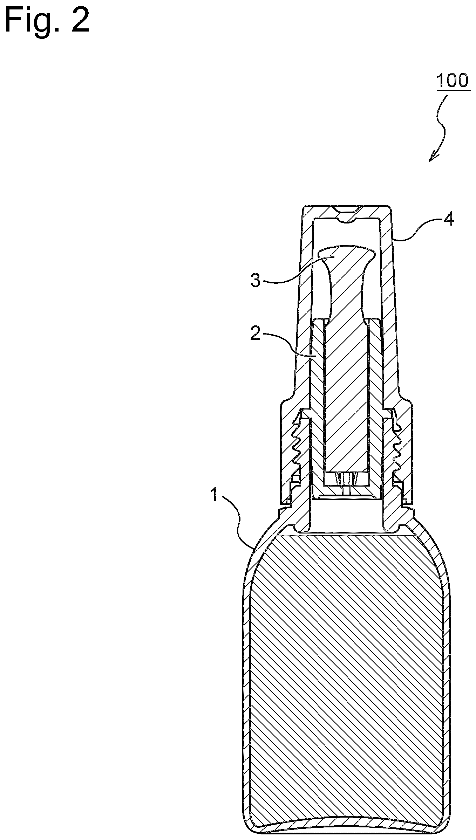

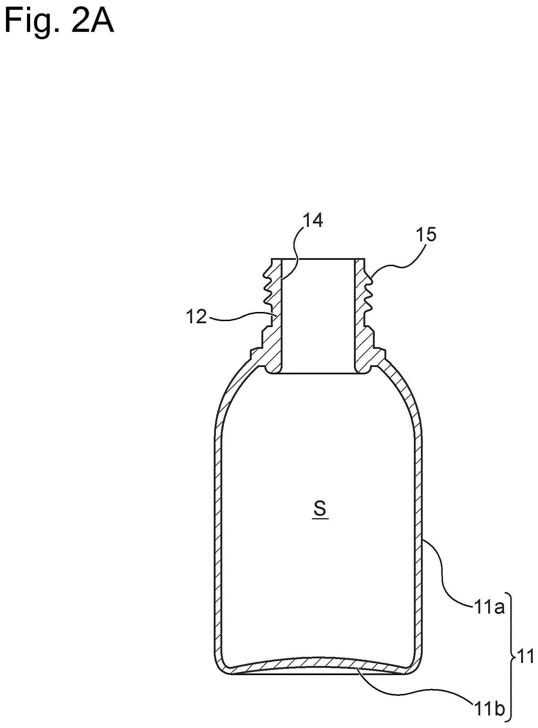

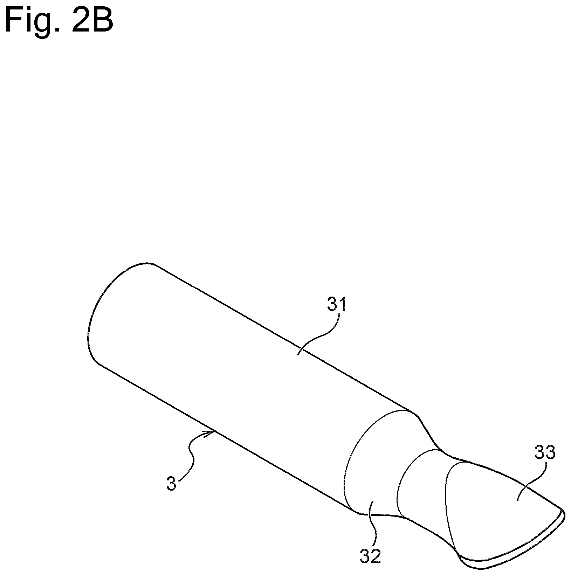

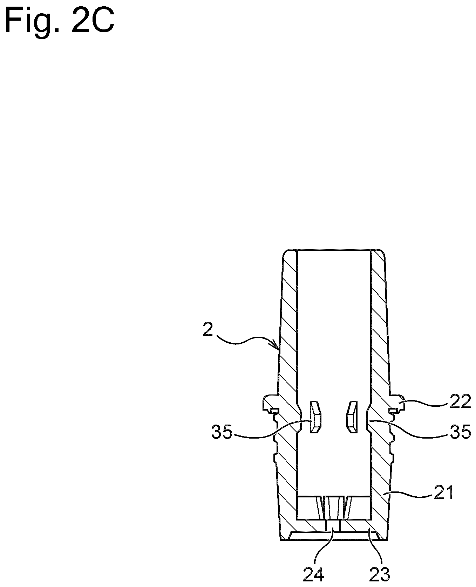

One embodiment of the present invention will be shown in Fig. 1 and Fig. 2. Fig. 1 is a schematic front view showing one example of an applicator including a liquid agent container according to one embodiment of the present invention, and Fig. 2 is a schematic cross-sectional view of the applicator shown in Fig. 1, the applicator including a cap that covers a columnar brush member. As shown in Fig. 1, an applicator 100 according to one embodiment of the present invention may be an applicator including a substantially cylindrical liquid agent container 1 in which a liquid agent can be loaded or filled, a bottomed tubular holder 2, and a columnar brush member 3. In addition, as shown in Fig. 2, the applicator 100 may include a detachable cap 4 that covers the brush member 3.

Fig. 2A is a schematic cross-sectional view showing the liquid agent container 1 alone. The liquid agent container 1 includes a liquid agent container main body 11 and a liquid agent container neck portion 12. In the example shown in the drawing, the liquid agent container main body 11 has a substantially cylindrical shape, and the upper portion is molded as a curved portion to be continuous with the liquid agent container neck portion 12. The liquid agent container neck portion 12 has an opening portion 14 in which the columnar brush member 3 is installed through the bottomed tubular holder 2 (refer to Fig. 2). On the outer surface of the liquid agent container neck portion 12, a screw thread 15 that can be screwed with the cap 4 is provided.

The liquid agent container main body 11 includes a side wall 11a that extends in the circumferential direction of the substantially cylindrical liquid agent container main body 11 and a bottom wall 11b. A loading space S in which the liquid agent can be loaded is defined by the side wall 11a and the bottom wall 11b. The applicator is placed in a state where the outside surface of the bottom wall 11b is in contact with the flat surface of a desk or the like while not in use or stored. A user pinches the side wall 11a when using the applicator 100.

Fig. 2B is a schematic perspective view for describing the columnar brush member 3. The columnar brush member 3 is a member formed in a columnar shape by bundling synthetic fibers. The columnar brush member 3 includes a columnar brush member main body 31, a columnar brush member neck portion 32, and a columnar brush member tip portion 33. The outer surfaces of the columnar brush member main body 31 and the columnar brush member neck portion 32 are in a state of being solidified with an adhesive, and synthetic fibers composed of fine polyester that are contained in the columnar brush member 3 are integrated. The columnar brush member tip portion 33 is formed in a brush shape by loosening synthetic fiber bundles of the tip portion of the columnar brush member neck portion 32 fixed with an adhesive. In the columnar brush member main body 31 and the columnar brush member neck portion 32, there are voids, which pass a liquid medicine, between the synthetic fibers, and a structure in which when an end surface of the columnar brush member main body 31 is immersed in a liquid medicine, the liquid medicine seeps out from the other ends of the synthetic fibers by the capillary action is thus formed.

Fig. 2C is a schematic cross-sectional view for describing the bottomed tubular holder 2. The bottomed tubular holder 2 includes a bottomed tubular holder main body 21 and an annular flange portion 22. The bottomed tubular holder main body 21 has a tubular shape. The tubular shape can be changed as appropriate along the outer circumferential shape of the columnar brush member 3 so that the columnar brush member 3 can be inserted thereinto. As shown in Fig. 2C, the bottomed tubular holder 2 has a bottom portion 23, and the bottom portion 23 and the bottomed tubular holder main body 21 having a tubular shape are joined to each other with no gap therebetween. A pore 24 is provided in the center of the bottom portion 23. The liquid medicine that has entered the inside of the liquid agent container 1 is capable of moving into the bottomed tubular holder 2 through this pore 24. In Fig. 2C, a reference sign 35 is a support member that is formed on the inner portion of the bottomed tubular holder main body 21 and supports the columnar brush member 3.

However, the specific shape of each component of the applicator 100 according to one embodiment of the present invention is not limited to that shown in the drawings.

An applicator according to one embodiment of the present invention is inverted during use. At this time, the brush member is supplied with the liquid agent from the liquid agent container at one end portion, and the liquid agent permeates into the brush member by the capillary action. The other end portion of the brush member functions as an application member for applying the liquid agent to nails. As described above, the liquid agent is supplied to nails through the brush member installed in the opening portion of the liquid agent container.

The user inverts the applicator according to one embodiment of the present invention (therefore, the liquid agent container) and is thereby capable of rapidly permeating the liquid agent into the brush member from the opening portion of the liquid agent container and smoothly applying an appropriate amount of the liquid agent to nails. Therefore, the applicator and liquid agent container according to one embodiment of the present invention have a preferable sensation of use.

In one embodiment of the present invention, the liquid agent container may have any form as long as the container allows liquid agents to be loaded therein with no leakage but is preferably, for example, a substantially cylindrical container having an opening portion in the upper portion as shown in Fig. 2A.

A main material of the liquid agent container according to one embodiment of the present invention is not particularly limited as long as the material is a synthetic resin that is suitable for loading medicines in the liquid agent container; however, as a base resin, polyethylene terephthalate, polybutylene terephthalate, polyethylene, polystyrene, polypropylene, or vinyl chloride is preferable, and polyethylene is particularly preferable.

In one embodiment of the present invention, the liquid agent container is not elastically deformable in normal actual usage, that is, an inelastic or inflexible and hard synthetic resin container. Users do not need to adjust the ejection amount of a liquid agent in a hard synthetic resin container with the pinching force. The liquid agent passively spreads in the brush member by the capillary action. Therefore, the ejection amount is less likely to vary with users, and the liquid agent container is a preferable container from such a viewpoint.

The internal volume of the liquid agent container in one embodiment of the present invention is not particularly limited but is preferably 8 mL to 12 mL.

The thickness of the side wall of the liquid agent container in one embodiment of the present invention is not particularly limited but is preferably 0.8 mm to 1.0 mm to comprehensively achieve the objects of the present invention.

In the present specification, "shielding the rays of light" refers to preventing the transmission of light using a substance that scatters light or a substance that absorbs light.

In the present invention, specific means for shielding the rays of light in the ultraviolet region is not particularly limited; however, for example, means as described below can be adopted using a substance capable of shielding ultraviolet rays in a predetermined wavelength range to be described below.
a) A method in which a substance shielding the rays of light in the ultraviolet region is added to the synthetic resin that is used for the container (for example, a method in which a substance shielding ultraviolet rays, such as titanium oxide, is added to the base resin and the base resin is molded into a container shape).
b) A method in which a member (for example, a film or the like) containing a substance shielding ultraviolet rays is separately attached to the surface of the container (for example, a method in which a shrink film with which an ultraviolet scattering agent has been blended is attached to the container).
c) A method in which a substance shielding ultraviolet rays is applied to the surface of the container.

Among the above-described means, b) and c) are not economical since the number of manufacturing steps or members increases. On the other hand, a) is preferable since manufacturing steps are simple, but it is not easy to shield only the ultraviolet rays and guarantee the visibility. In addition, when a selected container material is not appropriate, there is a possibility that an ingredient of the container material may elute into a content liquid.

In one embodiment of the present invention, a preferable liquid agent container is formed to shield ultraviolet rays by the method in which a substance shielding the ultraviolet rays is added to the synthetic resin that is used for the container. Therefore, a preferable liquid agent container in one embodiment of the present invention is a container composed of a single layer containing a substance shielding the ultraviolet rays uniformly dispersed in the main ingredient of the container material.

Here, "a substance shielding the ultraviolet rays" refers to a substance blocking the ultraviolet rays, preferably, titanium (IV) oxide. That is, "a container composed of a single layer containing a substance shielding the ultraviolet rays uniformly dispersed in the main ingredient of the container material" refers to a container molded in a state where a coloring ingredient, such as titanium (IV) oxide, is uniformly dispersed in the base resin.

The preferable liquid agent container in one embodiment of the present invention is composed of a single thermoplastic synthetic resin layer and is not composed of a plurality of layers. In addition, the container does not have any portion free of an ultraviolet blocking substance (or a portion having a lower concentration of an ultraviolet blocking substance than other portions) in a part of the container side surface. Such a portion is capable of functioning as a window portion or gauge portion for visually recognizing the quantity, but the preferable liquid agent container in one embodiment of the present invention does not include such a window portion or gauge portion.

In one embodiment of the present invention, the preferable liquid agent container can be molded by one molding step since material ingredients that are used for the liquid agent container have been uniformly dispersed one another. Therefore, the liquid agent container in one embodiment of the present invention can be manufactured by a simple and low-cost manufacturing method.

In one embodiment of the present invention, the preferable liquid agent container is a synthetic resin container that is not packaged with an ultraviolet absorbing film. Examples of an ultraviolet absorbent that is used in the film include salicylic acids, benzophenones, triazines, benzotriazoles, cyanoacrylates, and the like. The ultraviolet absorbing film may contain, for example, a shrinkable PET resin as a base material. When such an ultraviolet absorbing film is shrunk to cover the surface of the liquid agent container, it is possible to obtain the same effect as that of a synthetic resin container with which an ultraviolet shielding agent has been blended.

However, in the case of using the ultraviolet absorbing film, there is a possibility that a user may partially or fully damage the ultraviolet absorbing film by mistake or intentionally and the photostability of the content may be affected unintentionally. In addition, the use of the ultraviolet absorbing film may also increase the manufacturing cost. Therefore, particularly in pharmaceutical containers, it is preferable to form a single layer containing an ultraviolet shielding substance uniformly dispersed in a container material by adding the ultraviolet shielding substance to the material of the liquid agent container instead of using the ultraviolet absorbing film.

The liquid agent container of the present invention is a white (or semitransparent) synthetic resin container containing titanium (IV) oxide as a coloring ingredient. Titanium (IV) oxide functions as the ultraviolet shielding substance.

In one embodiment of the present invention, the preferable liquid agent container is a white synthetic resin container containing titanium (IV) oxide but not containing any coloring ingredients other than titanium (IV) oxide. In such a liquid agent container according to one embodiment of the present invention, an ingredient in the material of the container does not elute into the content liquid, which is preferable.

In the case of manufacturing the liquid agent container of the present invention, a method for adding titanium (IV) oxide is not particularly limited. For example, a predetermined amount of titanium (IV) oxide may be directly blended with the base material and molded. In addition, a masterbatch method is also preferable. In the case of the masterbatch method, for example, a masterbatch (MB) containing titanium (IV) oxide at a high concentration is produced by melting and/or kneading titanium (IV) oxide in a small amount of a resin in advance. Titanium (IV) oxide can be diluted to a predetermined concentration by mixing this masterbatch with the base resin. In this case, there is no limitation on masterbatches that can be used, and examples thereof include POLYCOOL MASTER: EPH-W3380 (manufactured by POLYCOL Co., Ltd.) and the like.

Regarding the light transmittance in the ultraviolet region of the liquid agent container of the present invention, from the viewpoint of suppressing the degradation of efinaconazole, the transmittance for light having wavelengths of 200 nm to 360 nm is all preferably 0.20% or less and more preferably 0.10% or less.

In order to realize a high light shielding effect in the ultraviolet region as described above, the lower limit amount of titanium (IV) oxide that is contained in the container of the present invention can be set.

In one embodiment of the present invention, the lower limit of the content of titanium (IV) oxide that is contained in a polyethylene liquid agent container having a thickness of 0.8 mm to 1.0 mm is preferably 0.09 parts by weight, more preferably 0.10 parts by weight, still more preferably 0.11 parts by weight, and far still more preferably 0.18 parts by weight relative to 100 parts by weight of the synthetic resin.

In the present specification, 100 parts by weight of the synthetic resin represents the total weight of materials that form the container of the present invention, such as the base resin, titanium (IV) oxide or the masterbatch, and the like.

Meanwhile, regarding the light transmittance in the visible light region of the liquid agent container according to one embodiment of the present invention, from the viewpoint of the visibility of the remaining amount of the liquid agent in the container, the transmittance for light having a wavelength of 700 nm is preferably 0.25% or more and more preferably 0.40% or more.

In another embodiment of the present invention, regarding the light transmittance in the visible light region of the liquid agent container, the transmittance for light having wavelengths of 700 nm to 780 nm is all preferably 0.25% or more and more preferably 0.40% or more.

In order to guarantee the light transmittance in the visible light region as described above, the upper limit amount of titanium (IV) oxide that is contained in the container of the present invention can be set.

In one embodiment of the present invention, the upper limit of the content of titanium (IV) oxide in the polyethylene liquid agent container having a thickness of 0.8 mm to 1.0 mm is preferably 0.33 parts by weight, more preferably 0.30 parts by weight, still more preferably 0.27 parts by weight, and far still more preferably 0.22 parts by weight relative to 100 parts by weight of the synthetic resin.

Therefore, one of the preferable embodiments of the liquid agent container of the present invention is a synthetic resin container for which the transmittance for light having wavelengths of 200 nm to 360 nm is all 0.20% or less and the transmittance for light having a wavelength of 700 nm is 0.25% or more.

One of the preferable different embodiments of the liquid agent container of the present invention is a synthetic resin container for which the transmittance for light having wavelengths of 200 nm to 360 nm is all 0.20% or less and the transmittance for light having wavelengths of 700 nm to 780 nm is all 0.25% or more.

One of the preferable different embodiments of the liquid agent container of the present invention is a synthetic resin container for which the transmittance for light having wavelengths of 200 nm to 360 nm is all 0.10% or less and the transmittance for light having a wavelength of 700 nm is 0.25% or more.

One of the preferable different embodiments of the liquid agent container of the present invention is a synthetic resin container for which the transmittance for light having wavelengths of 200 nm to 360 nm is all 0.10% or less and the transmittance for light having wavelengths of 700 nm to 780 nm is all 0.25% or more.

One of the preferable different embodiments of the liquid agent container of the present invention is a synthetic resin container for which the transmittance for light having wavelengths of 200 nm to 360 nm is all 0.20% or less and the transmittance for light having a wavelength of 700 nm is 0.40% or more.

One of the preferable different embodiments of the liquid agent container of the present invention is a synthetic resin container for which the transmittance for light having wavelengths of 200 nm to 360 nm is all 0.20% or less and the transmittance for light having wavelengths of 700 nm to 780 nm is all 0.40% or more.

One of the preferable different embodiments of the liquid agent container of the present invention is a synthetic resin container for which the transmittance for light having wavelengths of 200 nm to 360 nm is all 0.10% or less and the transmittance for light having a wavelength of 700 nm is 0.40% or more.

One of the preferable different embodiments of the liquid agent container of the present invention is a synthetic resin container for which the transmittance for light having wavelengths of 200 nm to 360 nm is all 0.10% or less and the transmittance for light having wavelengths of 700 nm to 780 nm is all 0.40% or more.

One of the preferable embodiments of the liquid agent container of the present invention is a synthetic resin container containing 0.09 to 0.33 parts by weight of titanium (IV) oxide relative to 100 parts by weight of the synthetic resin.

One of the preferable embodiments of the liquid agent container of the present invention is a synthetic resin container containing 0.10 to 0.30 parts by weight of titanium (IV) oxide relative to 100 parts by weight of the synthetic resin.

One of the preferable embodiments of the liquid agent container of the present invention is a synthetic resin container containing 0.11 to 0.27 parts by weight of titanium (IV) oxide relative to 100 parts by weight of the synthetic resin.

One of the preferable embodiments of the liquid agent container of the present invention is a synthetic resin container containing 0.18 to 0.22 parts by weight of titanium (IV) oxide relative to 100 parts by weight of the synthetic resin.

The liquid agent container according to one embodiment of the present invention as described above is a container that achieves two conflicting objects. That is, according to one embodiment of the present invention, it is possible to provide a liquid agent container enabling both the degradation of efinaconazole due to ultraviolet rays to be suppressed and the remaining amount of a content to be visually recognized easily by elaborately controlling the light transmittance for light both in the ultraviolet region and in the visible light region.

"The transmittance for light having wavelengths of X nm to Y nm" in the present specification means the light transmittance at one arbitrary wavelength selected from the entire wavelength range of X nm to Y nm.

In addition, the light transmittance can be measured using, for example, a commercially available spectrophotometer (U-3310: manufactured by Hitachi, Ltd.). In addition, "light transmittance spectrum" refers to a spectrum in which light transmittance in a measured arbitrary wavelength range is plotted as a continuous spectrum.

The configuration of the brush member that the applicator of the present invention includes is not particularly limited as long as the effect of the present invention can be obtained, but a brush member having a configuration described with reference to Fig. 2B is preferable.

Specifically, in one embodiment of the present invention, a preferable brush member is a columnar brush member configured by bundling a plurality of synthetic fibers composed of polyester.

A preferable brush member in a different embodiment of the present invention is a columnar brush member configured by bundling a plurality of synthetic fibers composed of polyethylene or nylon.

The synthetic fiber that is used in the present invention is preferably a synthetic fiber having fiber diameter within a range of 7 µm to 50 µm and more preferably a synthetic fiber having fiber diameter within a range of 10 µm to 30 µm.

In addition, the synthetic fiber that is used in the present invention may be glued with an adhesive.

The density of the adhesive gluing the synthetic fiber is preferably within a range of 0.15 to 0.65 (porosity: 85% to 35%) and more preferably within a range of 0.25 to 0.50 (porosity: 75% to 50%).

In the present specification, the density means the proportion of the synthetic fibers and the adhesive gluing the same per unit cross-sectional area based on a cross section cut perpendicular to the direction of the fibers in the brush member.

In a case where the density of the synthetic fiber is lower than 0.15, there are many flow paths (voids) for a liquid medicine, and an excess amount of the liquid is thus ejected when the container is inverted to eject the liquid. In addition, since the proportion of the portion of a structure is small, it becomes difficult to maintain the strength of the columnar brush member main body, and the brush member is likely to break.

On the other hand, in a case where the density of the synthetic fiber is higher than 0.65, the number of the flow paths (voids) for a liquid medicine decreases, the liquid medicine is less likely to permeate, and smooth application of the liquid medicine is hindered.

The volume of the preferable brush member relies on the volume of the liquid agent that permeates by the capillary action and is held in the columnar brush member, the viscosity of the liquid agent, or the like. When the viscosity of the liquid agent that is used in the present invention is low, it is preferable to increase the volume of the columnar brush member. In one embodiment of the present invention, the volume of the preferable brush member is within a range of 400 mm³ to 600 mm³.

A brush member described in Examples of the present invention, which will be described below, is a brush member obtained by shaping polyethylene fiber having a fiber diameter of 18 µm and a fineness of 3.3 dtex in a columnar shape with a density of 0.42 (porosity: 58%). The volume of the columnar brush member used in the present Examples is 490 mm³.

A brush member in a different embodiment of the present invention may be a calligraphy-brush-like brush member obtained by welding one end of the bundle of synthetic fibers.

In one embodiment of the present invention, the applicator may further include a bottomed tubular holder. The configuration of the bottomed tubular holder that the applicator according to one embodiment of the present invention includes is not particularly limited as long as the effect of the present invention can be obtained, but a bottomed tubular holder having a configuration described with reference to Fig. 2C is preferable.

The bottomed tubular holder is used to hold the brush member and reliably connect the brush member to the liquid agent container.

The bottomed tubular holder has a tubular shape, and the brush member is inserted thereinto. In this case, the inner surface of the bottomed tubular holder and the outer surface of the brush member are in close contact with each other, and there is no gap in the configuration. In addition, the outer surface of the bottomed tubular holder is liquid-tightly fitted into the opening portion of the liquid agent container with no gap therebetween.
Thus, the occurrence of liquid leakage can be prevented.

A pore is provided in the bottom portion of the bottomed tubular holder, and the pore functions as a flow path for the liquid agent toward the brush member from the liquid agent container.

In one embodiment of the present invention, when the applicator is held with the brush member side downward, the liquid agent loaded in the liquid agent container permeates into the bottomed tubular holder through the pore and reaches one end surface (on a side where the end surface is supported by the bottomed tubular holder) of the brush member. The liquid agent that has reached the end surface of the brush member reaches the other end surface (on a side where the liquid agent is applied to nails) of the brush member by the capillary action. This makes it possible to apply the liquid agent to nails.

The ejection amount of the liquid agent can also be increased or decreased by the shape or size of the pore provided in the bottom portion of the bottomed tubular holder. The position, number, shape, and size of the pore can be set as appropriate depending on the properties, such as viscosity, of the liquid medicine to be used. For example, aside from a circular shape, shapes such as an elliptical shape, a polygonal shape, and a parallelogram shape can be selected depending on the purpose or the use.

In addition, regarding the size of the pore, the maximum diameter is preferably within a range of 0.5 mm to 5 mm and more preferably within a range of 0.9 mm to 1.3 mm based on a surface parallel to the bottom portion of the bottomed tubular holder.

An applicator described in Examples of the present specification, which will be described below, includes a bottomed tubular holder having the above-described features, and one pore is provided in the center of the bottom portion and has a circular shape (diameter: 1.1 mm).

Regarding the applicator of the present invention, the expression "favorable sensation of use" refers to the fact that a user is able to smoothly apply the right amount of the liquid agent to the entire infected nails when ejecting the liquid agent from the applicator. For example, in a case where a single dose of the liquid agent can be appropriately applied without being affected by the usage environment (for example, the temperature) and/or the user's pinching force, the sensation of use of the applicator is favorable.

### <2> Liquid agent

"Liquid agent" in the present invention refers to an agent obtained by dissolving, emulsifying, or suspending an active ingredient and an additive or the like in a solvent. The liquid agent container and applicator of the present invention are applied to liquid agents containing efinaconazole as an active ingredient. The liquid agent container and applicator of the present invention are used for the treatment of tinea unguium by applying an efinaconazole liquid agent to the entire infected nails once a day.

One embodiment of "liquid agent containing efinaconazole" is a liquid agent obtained by adding and dissolving efinaconazole and one or more pharmaceutically acceptable additives as necessary to and in water, an organic solvent, or a solvent mixture of water and an organic solvent.

In the present invention, the content of efinaconazole is 10% relative to the total weight of the liquid agent.

Pharmaceutically acceptable additives may be blended with the liquid agent that is loaded into the container of the present invention as necessary. The additives may be antioxidants and may be preferably dibutylhydroxytoluene (BHT) and ethylenediaminetetraacetic acid (EDTA).

As a solvent that is used for a liquid agent that is loaded into the container of the present invention, water, an organic solvent, or a solvent mixture of water and an organic solvent can be used. Examples of the organic solvent that can be used include ethanol, propylene glycol, glycerin, triacetin, isopropanol, isopropyl adipate, alkyl lactate, cyclomethicone, and solvent mixtures obtained by mixing two or more of these organic solvents.

A preferable solvent in the present invention is ethanol. Therefore, a preferable liquid agent as a liquid agent that is loaded into the container of the present invention is a formulation of an ethanol solution in which the content of efinaconazole is 10% relative to the total weight of the liquid agent.

### EXAMPLES

Hereinafter, the present invention will be more specifically described with Examples, but the present invention is not limited only to the following Examples.

### <Manufacturing Example>

Each liquid agent container was manufactured according to Table 1. Specifically, a predetermined amount of MB (masterbatch: POLYCOOL MASTER, EPH-W3380) was added to a base resin (NOVATEC (Registered Trademark) HD, HB332R) and melt-kneaded, and each container was then manufactured by blow molding. Each liquid agent container was inflexible and hard. In addition, as the container thickness, the thickness of the side wall of the container was measured.

An efinaconazole 10% solution was loaded into each container, a bottomed tubular holder having a brush member inserted thereinto was fitted into the container, and a cap was tightened, thereby manufacturing each applicator. As the efinaconazole 10% solution, a liquid agent composed of 0.00025 wt% of disodium EDTA, 1.00 wt% of purified water, 0.10 wt% of anhydrous citric acid, 0.10 wt% of BHT, 10.00 wt% of C12-15 alkyl lactate, 12.00 wt% of diisopropyl adipate, 13.00 wt% of cyclomethicone, 10.00 wt% of efinaconazole, and an appropriate amount (an amount by which the total reached 100.00 wt%) of 95% ethanol was used.

**[Table 1]**

| Container No. | Container thickness (mm) | MB blending amount (%)^{*1} | Net content of titanium (IV) oxide(%) | Light transmittance at 360 mm (%)^{*2} | Light transmittance at 700 nm (%) |
|---|---|---|---|---|---|
| Comparative Example 1 | 0.7 | 0.5% | 0.09 to 0.11 | 0.87 | 8.60 |
| Comparative Example 2 | 0.7 | 5.0% | 0.9 to 1.1 | -0.01 | 0.19 |
| Comparative Example 3 | 0.9 | 2.0% | 0.36 to 0.44 | -0.01 | 0.23 |
| Compara tive Example 4 | 0.9 | 5.0% | 0.9 to 1.1 | -0.01 | 0.13 |
| Example 1 | 0.7 | 1.0% | 0.18 to 0.22 | 0.04 | 1.14 |
| Example 2 | 0.9 | 0.5% | 0.09 to 0.11 | 0.07 | 2.14 |
| Example 3 | 0.9 | 1.0% | 0.18 to 0.22 | -0.01 | 0.44 |
| Example 4 | 0.9 | 1.5% | 0.27 to 0.33 | -0.01 | 0.28 |
| Example 5 | 1.0 | 1.0% | 0.18 to 0.22 | -0.01 | 0.35 |

| | | | | | |
|---|---|---|---|---|---|
| *1 MB (masterbatch): POLYCOOL MASTER, EPH-W3380 (manufactured by POLYCOL Co., Ltd.) *2 The transmittance of the container for light of 200 nm to 360 nm in the table all indicates the maximum value at 360 nm. | | | | | |

### <Test Example 1>

### <Photostability test>

A photostability test was performed on, among the containers shown in Table 1, the container of each of Comparative Example 1 and 2 and Example 1 to 3. These containers are a variety of containers that are different in the container thickness and the content of titanium (IV) oxide. Four milliliters of the efinaconazole 10% solution was loaded into each of the containers, a bottomed tubular holder having a brush member inserted thereinto was fitted into the container, and specimens tightened with caps were arranged while lying on their sides in a photostability tester and stored under the following conditions. As the photostability tester, "photostability test-oriented stability tester" (LTL-200A-14WCD: manufactured by Nagano Science Co., Ltd.) was used.
Light source: D65 fluorescent lamp
Illuminance: 2000 lux
Temperature: 25°C ± 2°C
Humidity: 60%RH ± 5%RH

The specimens were removed and the qualities thereof were evaluated at the beginning of the test (before irradiation with the D65 fluorescent lamp) and when approximately 1,200,000 lux·hr (25 days) was reached. The amounts of impurities were analyzed by high performance liquid chromatography. From the chromatograms of sample solutions, the area of each peak was measured by an automatic integration method, and the amounts thereof were obtained by an area normalization method. For three containers of each of Comparative Examples 1 and 2 and Examples 1 to 3, the maximum peaks out of the peaks of unknown impurities that were newly generated in the specimens were specified, and the average value of the amounts was calculated.

In the photostability test in the present specification, in a case where the maximum value of the peaks of unknown impurities that were newly generated upon 1,200,000 lux·hr irradiation was 0.20% or less, the photostability was determined to be guaranteed, and A was entered into the "Photostability" column (A evaluation). On the other hand, in a case where the maximum value described above exceeded 0.20%, the photostability was determined not to be guaranteed, and B was entered into the "Photostability" column (B evaluation). The results are shown in Table 2.

**[Table 2]**

| Container No. | Container thickness (mm) | Net content of titanium (IV) oxide (%) | Maximum value of light transmittance within range of 200 nm to 360 nm (%) | Maximum value of peak of unknown impurity generated upon 1,200,000 lux·hr irradiation (%) | Photostability |
|---|---|---|---|---|---|
| Comparative Example 1 | 0.7 | 0.09 to 0.11 | 0.87 | 0.28 (RRT^{*}:1.48) | B |
| Comparative Example 2 | 0.7 | 0.9 to 1.1 | -0.01 | 0.03 (RRT^{*}:1.13) | A |
| Example 1 | 0.7 | 0.18 to 0.22 | 0.04 | 0.10 (RRT^{*}:1.49) | A |
| Example 2 | 0.9 | 0.09 to 0.11 | 0.07 | 0.02 (RRT^{*}:1.50) | A |
| Example 3 | 0.9 | 0.18 to 0.22 | -0.01 | 0.08 (RRT^{*}:1.50) | A |

| | | | | | |
|---|---|---|---|---|---|
| * RRT (relative retention time): Relative retention time relative to the holding time of efinaconazole | | | | | |

In Comparative Example 1, the maximum value of the peaks of unknown impurities that were newly generated upon 1,200,000 lux·hr irradiation was 0.28%, and the container failed to satisfy the evaluation standard of photostability in the present test (B evaluation). In Comparative Example 2 and Examples 1 to 3, the containers satisfied the evaluation standard of photostability (A evaluation). In addition, in Examples 1 to 3, the total amounts of the impurities upon 1,200,000 lux·hr irradiation were 0.08% to 0.15%. These results show that the containers of Examples 1 to 3 are containers capable of stably storing efinaconazole liquid agents.

As is clear from Table 2, the transmittance for light having wavelengths of 200 nm to 360 nm decreased along with an increase in the content of titanium (IV) oxide. In addition, the transmittance for light having wavelengths of 200 nm to 360 nm decreased along with an increase in the thickness of the container.

In Comparative Example 2, Example 1, Example 2, and Example 3, it was possible to stably store the efinaconazole liquid agents. On the other hand, in Comparative Example 1, it was confirmed that it was not possible to suppress an increase in the amount of the impurity derived from efinaconazole. From these facts, it became clear that the photostability of the efinaconazole liquid agent loaded into each container relies on the transmittance for light having wavelengths of 200 nm to 360 nm, and in a case where the transmittance for light having wavelengths of 200 nm to 360 nm is all 0.20% or less, it is possible to stably store the efinaconazole liquid agent.

From these results, it was confirmed that the transmittance for light having wavelengths of 200 nm to 360 nm is important for an increase in the amount of an impurity in the efinaconazole liquid agent due to light exposure and the degradation of efinaconazole can be suppressed by shielding the rays of light in such a wavelength region.

Here, in Example 4 and Example 5 not shown in Table 2, ultraviolet rays are scarcely transmitted as in Example 3 (Table 1). In addition, Example 4 is a container that has the same container thickness as Example 2 and Example 3 that satisfy the evaluation standard of the photostability test and has a larger content of titanium (IV) oxide than Example 2 and Example 3. In addition, Example 5 is a container that has the same content of titanium (IV) oxide as Example 1 and Example 3 that satisfy the evaluation standard of the photostability test and has a thicker side wall than Example 1 and Example 3. Therefore, the containers of Example 4 and Example 5 are understood as containers that are more stable against light and satisfy the evaluation standard of the photostability test in the present specification (A evaluation).

### <Test Example 2>

### <Light transmittance test>

The side wall of the container of each of Comparative Examples 1 to 4 and Examples 1 to 5 was cut in an approximately 1 cm × 2 cm rectangular shape using scissors. Each of the cut bottle pieces was installed on the light-emitting portion side of the cell holder of a spectrophotometer, and the transmittance spectrum at wavelengths of 200 to 800 nm was measured. The measurement results and the evaluation results of whether or not the remaining amount of the liquid agent can be visually recognized from the outside of the container when the container is loaded with the efinaconazole liquid agent are shown in Table 3.

**[Table 3]**

| Container No. | Container thickness (mm) | Net content of titanium (IV) oxide(%) | Light transmittance at 700 nm (%) | Minimum value of light transmittance within range of 700 nm to 780 nm (%) | Visibility of liquid agent |
|---|---|---|---|---|---|
| Comparative Example 1 | 0.7 | 0.09 to 0.11 | 8.60 | 8.60 | Visible |
| Comparative Example 2 | 0.7 | 0.9 to 1.1 | 0.19 | 0.19 | Not Visible |
| Comparative Example 3 | 0.9 | 0.36 to 0.44 | 0.22 | 0.22 | Not Visible |
| Comparative Example 4 | 0.9 | 0.9 to 1.1 | 0.13 | 0.13 | Not Visible |
| Example 1 | 0.7 | 0.18 to 0.22 | 1.14 | 1.14 | Visible |
| Example 2 | 0.9 | 0.09 to 0.11 | 2.14 | 2.14 | Visible |
| Example 3 | 0.9 | 0.18 to 0.22 | 0.44 | 0.44 | Visible |
| Example 4 | 0.9 | 0.27 to 0.33 | 0.28 | 0.28 | Visible |
| Example 5 | 1.0 | 0.18 to 0.22 | 0.35 | 0.35 | Visible |

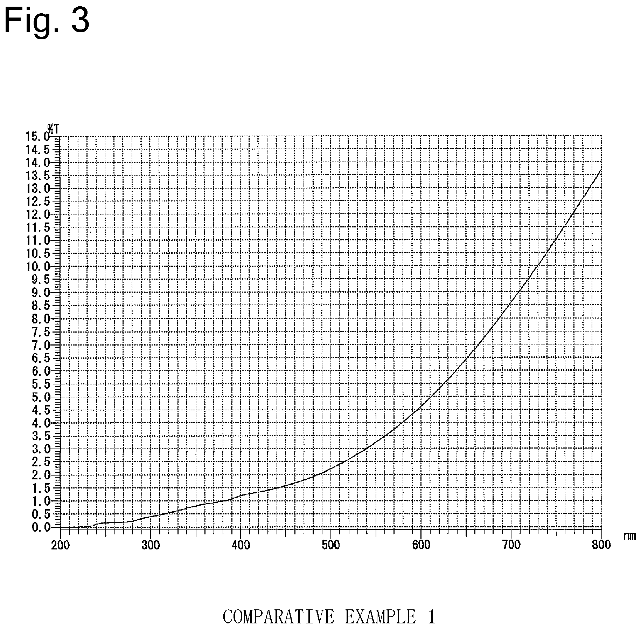

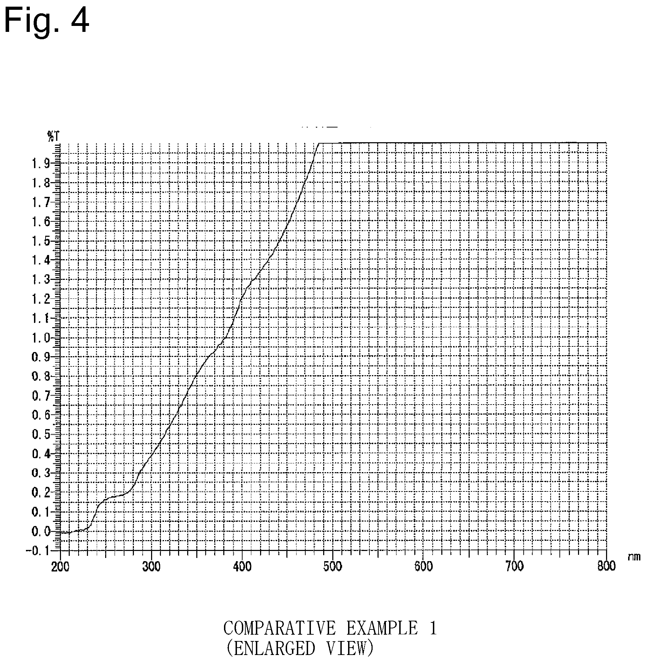

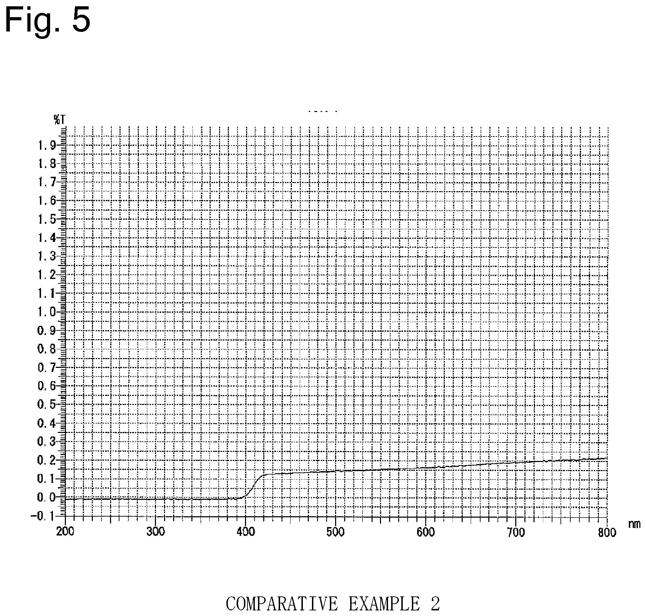

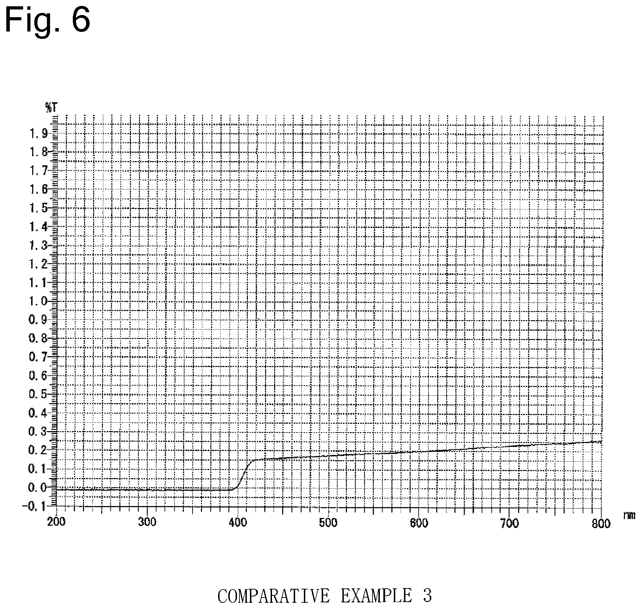

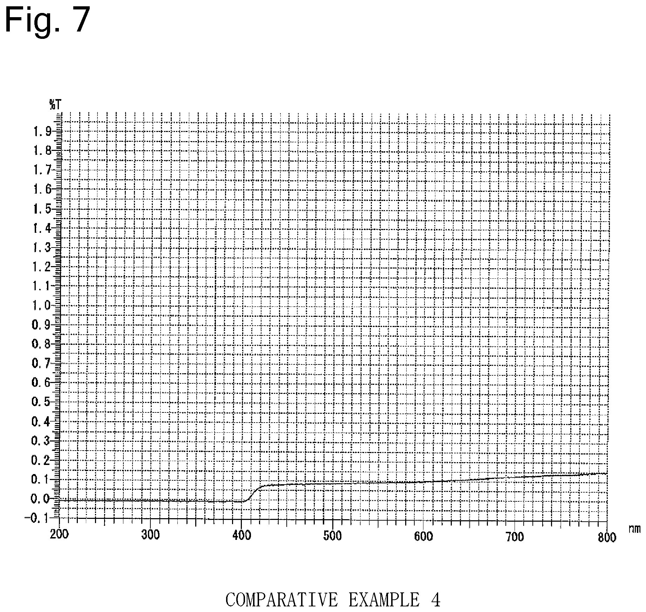

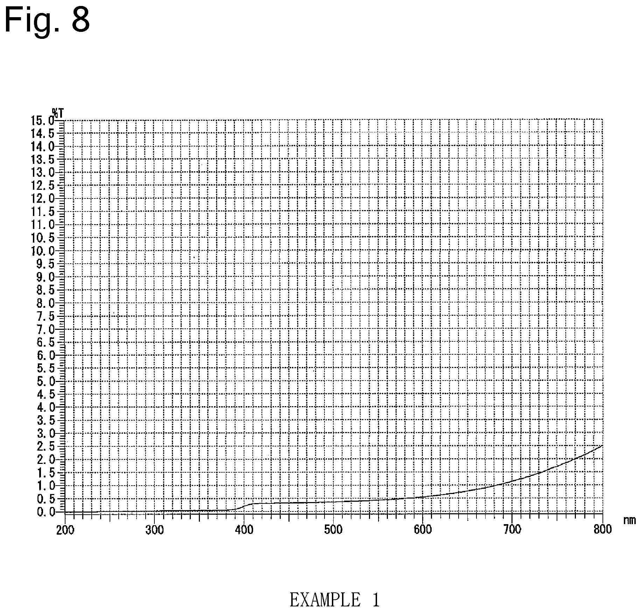

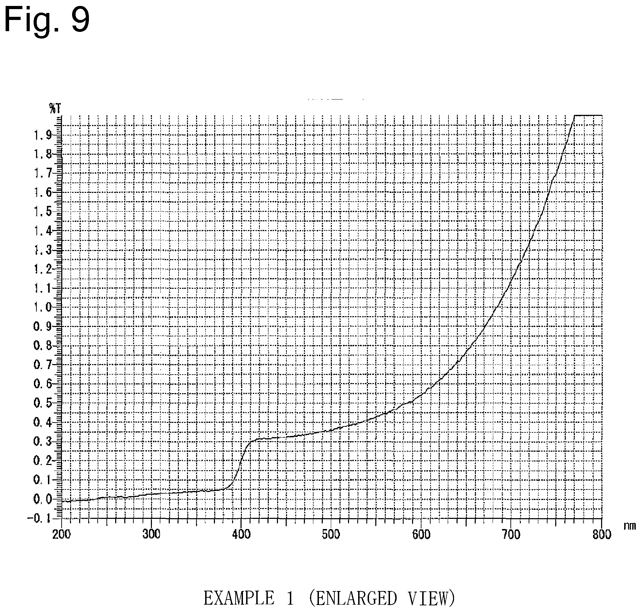

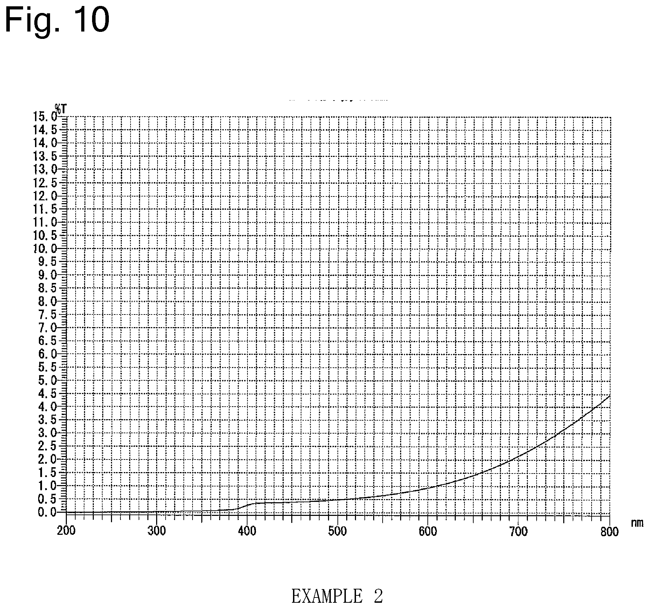

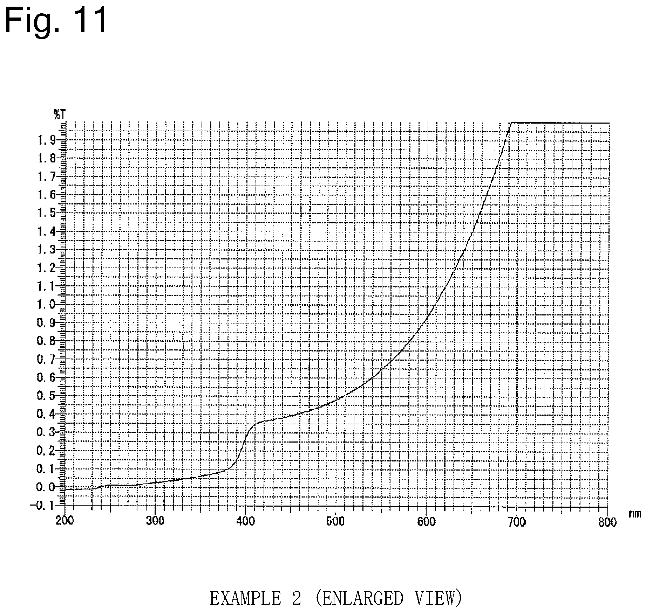

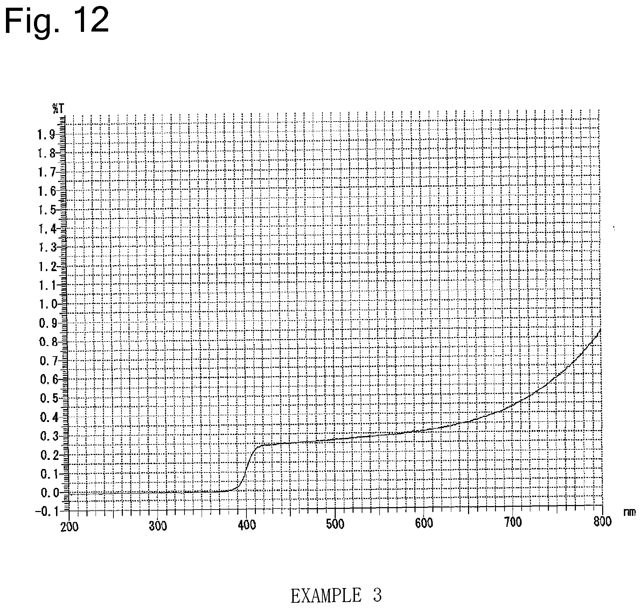

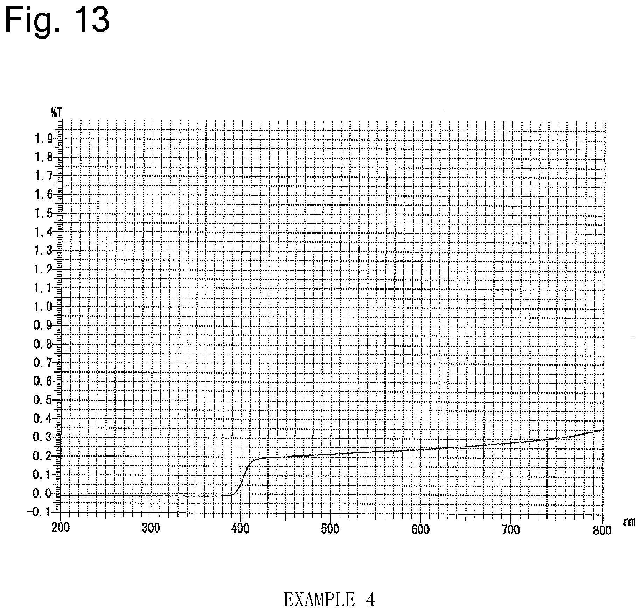

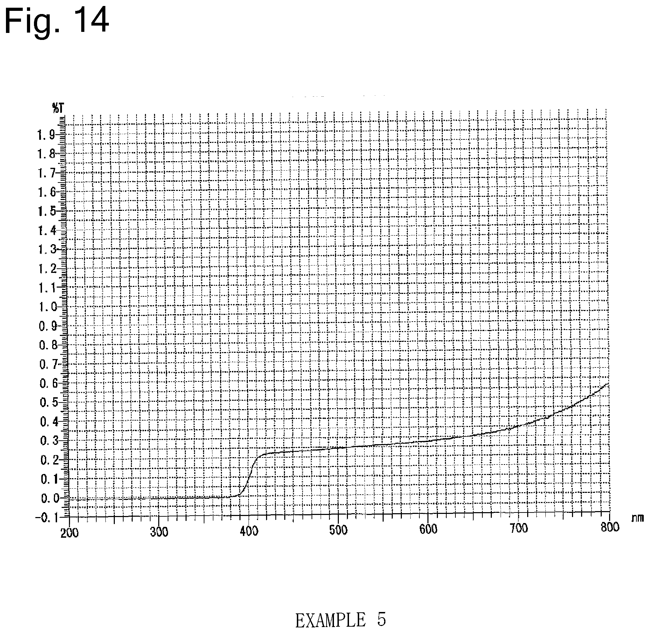

The containers used in the light transmittance test were also a variety of containers different in the container thickness and the content of titanium (IV) oxide, and nine kinds of containers shown in Table 3 were used. In addition, the transmittance of each container for light having a wavelength of 700 nm, the transmittance for light having wavelengths of 700 nm to 780 nm, and whether or not the amount of the liquid agent loaded into each container can be visually recognized from the outside are shown in Table 3. In Comparative Examples 2 to 4, it was difficult to visually recognize the liquid agents in the containers; however, in Comparative Example 1 and Examples 1 to 5, it was possible to visually recognize the liquid agents in the containers. For all of the containers, the transmittance spectra at wavelengths of 700 nm to 780 nm were on an upward trend, and the transmittance for light having a wavelength of 700 nm showed the minimum value in the range. In addition, the transmittance spectra of the individual containers of Comparative Examples 1 to 4 and Examples 1 to 5 are shown in Fig. 3 to Fig. 14. In Fig. 3 to Fig. 14, the horizontal axes indicate wavelengths (nm), and the vertical axes indicate light transmittance (%).

As is clear from Table 3, the transmittance for light having wavelengths of 700 nm to 780 nm decreased along with an increase in the content of titanium (IV) oxide. In addition, the transmittance for light having wavelengths of 700 nm to 780 nm decreased along with an increase in the thickness of the container.

The visibility of the efinaconazole liquid agent loaded into each container from the outside of the container was related to the transmittance for light having wavelengths of 700 nm to 780 nm, and in Comparative Example 1 and Examples 1, 2, 3, 4, and 5 where the transmittance for light having a wavelength of 700 nm was 0.25% or more, it was possible to visually recognize the content. On the other hand, in Comparative Examples 2, 3, and 4 where the transmittance for light having a wavelength of 700 nm was less than 0.25%, it was not possible to visually recognize the content.

From the above-described results, it was possible to confirm that for the containers enabling the efinaconazole liquid agent to be visually recognized, at least the transmittance for light having a wavelength of 700 nm needs to be 0.25% or more.

### <Test Example 3>

### <Application test>

Four milliliters of the efinaconazole 10% solution was loaded into the container of each of Comparative Example 2 and Example 3 shown in Table 1, a bottomed tubular holder having a brush member inserted thereinto was fitted into the container, and a specimen tightened with a cap was manufactured. After the mass of a bottle before the application of a medicinal solution was measured, the medicinal solution was applied to the entire surface of each of five 4 cm² stainless steel pieces. The above-described method was performed twice to apply the medicinal solution to 10 stainless steel pieces, and the mass of the bottle after the application of the medicinal solution was measured. The present operation was designed with an assumption that the medicinal solution was applied to 10 toenails. The application amount was calculated by subtracting the mass of the bottle after the application of the medicinal solution from the mass of the bottle before the application of the medicinal solution. During the application, the container was held as if the entire container was grasped, and the container was inclined during the application at three angles of 90°, 45°, and 10° to 20° with respect to the surface to be coated. The test was performed five times on each container at each application angle, and the average application amount and the standard deviation were calculated.

The results of the present test are shown in Table 4 below.

**[Table 4]**

| Container No. | Container thickness (mm) | Application angle (° ) | Application amount ± standard deviation (g) |
|---|---|---|---|
| Comparative Example 2 | 0.7 | 90 | 0.28±0.09 |
| | | 45 | 0.25±0.11 |
| | | 10 to 20 | 0.22±0.08 |
| Example 3 | 0.9 | 90 | 0.16±0.08 |
| | | 45 | 0.16±0.08 |
| | | 10 to 20 | 0.14±0.07 |

The application amounts were appropriate for all of the containers, and the applicators functioned as appropriate. When the application amounts in Comparative Example 2 and Example 3 are compared with each other, in Example 3, a tendency of suppressing the application amount is recognized. In addition, regarding the application angle, a tendency that the application amount increases as the inclination of the container approaches being perpendicular (90° ≥ 45° ≥ 10° to 20°) is recognized.

### <Test Example 4>

### <Ejection test (dropping time) and test of sensation of use>

In each of Comparative Example 2 and Example 3, five containers were prepared. Four milliliters of the efinaconazole 10% solution was loaded into each of these containers, a bottomed tubular holder having a brush member inserted thereinto was fitted into the container, and the container was subjected to the test.

Each container was fixed in an inverted state using a silicone tube connected to a constant-temperature water bath and then heated to 32°C, and the appearance of the liquid agent dropping was observed. The number of drops was counted, and a time taken for each dropping to occur was measured.

In Comparative Example 2 and Example 3, the times taken for the first dropping to occur from the beginning of the test were 22.4 seconds and 41.5 seconds, respectively. In addition, the times taken for the second dropping to occur from the first dropping were 2.6 seconds and 6.3 seconds, respectively. The numbers of the drops dropped until the liquid agent did not drop any more for one minute were 13 and 8, respectively.

As described above, when Comparative Example 2 (the thickness of the side wall: 0.7 mm) and Example 3 (the thickness of the side wall: 0.9 mm) are compared with each other, the time taken for the dropping is longer in the latter case.

Next, on Comparative Example 2 and Comparative Example 4, sensory evaluations of the sensation of use were performed by 80 panelists. As a result, it was evaluated in Comparative Example 4 that the liquid agent was not excessively discharged and more smooth liquid agent application was possible. Here, since the content of titanium (IV) oxide in the container material has no influences on the sensation of use, in Examples 2 to 4 where the thickness of the side wall is the same as that in Comparative Example 4, the sensation of use is considered to be as preferable as in Comparative Example 4.

As described above, Examples 1 to 5 are preferable since stable loading of the efinaconazole 10% solution is possible and the quantity can be visually recognized. Particularly, Examples 2 to 4 are more preferable from the viewpoint of the sensation of use.

### INDUSTRIAL APPLICABILITY

The container of the present invention shields the rays of light having wavelengths, which accelerate the photolysis of efinaconazole, while holding a preferable sensation of use and enables the remaining amount of a liquid agent loaded into the container to be visually recognized from the outside of the container and is thus useful as a container for loading liquid agents of efinaconazole.

### REFERENCE SIGNS LIST

S Loading space
1 Liquid agent container
2 Bottomed tubular holder
3 Columnar brush member
4 Cap
11 Liquid agent container main body
11a Side wall
11b Bottom wall
12 Liquid agent container neck portion
14 Opening portion
15 Screw thread
21 Bottomed tubular holder main body
22 Annular flange portion
23 Bottom portion
24 Pore
31 Columnar brush member main body
32 Columnar brush member neck portion
33 Columnar brush member tip portion
35 Support member
100 Applicator

## Claims

1. A liquid agent container into which a liquid agent containing 10% of efinaconazole can be loaded,
wherein the liquid agent container is a container formed from a synthetic resin containing titanium (IV) oxide,
transmittance for light having a wavelength of 200 nm to 360 nm is all 0.20% or less, transmittance for light having a wavelength of 700 nm is 0.25% or more, and a quantity of the liquid agent loaded into the liquid agent container is visually recognizable from outside.

2. The liquid agent container according to claim 1, wherein the liquid agent container is a substantially cylindrical polyethylene container having an opening portion in an upper portion, and the container is composed of a single layer containing at least titanium (IV) oxide uniformly dispersed in the polyethylene and is an inflexible and hard container.

3. The liquid agent container according to claim 1 or 2, wherein the liquid agent container has an internal volume of 8 mL to 12 mL and a thickness of 0.8 mm to 1.0 mm in a side wall of the liquid agent container.

4. The liquid agent container according to any one of claims 1 to 3, wherein a content of the titanium (IV) oxide is 0.09 to 0.33 parts by weight relative to 100 parts by weight of the synthetic resin.

5. The liquid agent container according to any one of claims 1 to 4, wherein a content of the titanium (IV) oxide is 0.10 to 0.30 parts by weight relative to 100 parts by weight of the synthetic resin.

6. The liquid agent container according to any one of claims 1 to 5, wherein a content of the titanium (IV) oxide is 0.18 to 0.22 parts by weight relative to 100 parts by weight of the synthetic resin.

7. The liquid agent container according to any one of claims 1 to 6, wherein no coloring ingredients other than the titanium (IV) oxide are contained.

8. The liquid agent container according to any one of claims 1 to 7, wherein transmittance of the liquid agent container for light having wavelengths of 700 nm to 780 nm is all 0.25% or more.

9. The liquid agent container according to any one of claims 1 to 8, wherein transmittance of the liquid agent container for light having wavelengths of 700 nm to 780 nm is all 0.40% or more.

10. The liquid agent container according to any one of claims 1 to 9, wherein transmittance of the liquid agent container for light having wavelengths of 200 nm to 360 nm is all 0.10% or less.

11. The liquid agent container according to any one of claims 1 to 10, wherein the liquid agent container is not packaged with an ultraviolet absorbing film.

12. An applicator comprising:
the liquid agent container according to any one of claims 1 to 11;
a columnar brush member shaped in a columnar shape by bundling synthetic fibers; and
a bottomed tubular holder having a tubular main body and a bottom portion between the liquid agent container and the columnar brush member,
wherein the bottomed tubular holder is liquid-tightly fitted into the opening portion of the liquid agent container,
the bottom portion of the bottomed tubular holder has at least one pore,
the tubular brush member is inserted into an inside of the tubular main body of the bottomed tubular holder, and
the liquid agent is allowed to pass from the liquid agent container to the tubular brush member through the pore, which makes it possible, when the liquid agent container is inverted during use of the applicator, for the liquid agent to be permeated into the tubular brush member and be applied to a user's nail.

13. The applicator according to claim 12, wherein the tubular brush member is shaped by bundling a synthetic fiber having a fiber diameter within a range of 7 µm to 50 µm at a density within a range of 0.25 to 0.50, and
the bottomed tubular holder has one circular pore having a diameter of 0.9 mm to 1.3 mm.

14. The applicator according to claim 12 or 13, wherein in a case where the applicator loaded with 4 mL of the liquid agent is inverted in an environment of 32°C, the number of drops dropped until the liquid agent does not drop any more for one minute is 7 to 10.

15. A photostabilization method of efinaconazole, comprising:
a step of loading a liquid agent containing 10% of efinaconazole into the liquid agent container according to any one of claims 1 to 11 or the applicator according to any one of claims 12 to 14.
